Europäisches Patentamt

European Patent Office

Office européen des brevets

(11) Veröffentlichungsnummer: **0 367 856 B1**

(12) **EUROPÄISCHE PATENTSCHRIFT**

(45) Veröffentlichungstag der Patentschrift: **03.02.93**

(21) Anmeldenummer: **88118681.1**

(22) Anmeldetag: **09.11.88**

(51) Int. Cl.5: **A61B 6/03**

(54) **Computer-Tomograph.**

(43) Veröffentlichungstag der Anmeldung:
**16.05.90 Patentblatt 90/20**

(45) Bekanntmachung des Hinweises auf die
Patenterteilung:
**03.02.93 Patentblatt 93/05**

(84) Benannte Vertragsstaaten:
**DE FR GB**

(56) Entgegenhaltungen:
**EP-A- 0 133 488**
**DE-A- 3 017 494**
**GB-A- 1 493 596**
**GB-A- 1 522 307**

**PATENT ABSTRACTS OF JAPAN, Band 3, Nr.**
**5, 18. Januar 1979, Seite 156 E84; & JP-A-53**
**132 985 (NIPPON DENSHI K.K.) 20-11-1978**

(73) Patentinhaber: **SIEMENS AKTIENGESELL-**
**SCHAFT**
**Wittelsbacherplatz 2**
**W-8000 München 2(DE)**

(72) Erfinder: **Schwierz, Günter, Dr.rer.nat.**
**Rödlaser Strasse 9**
**W-8524 Neunkirchen(DE)**

**Beschreibung**

Die Erfindung betrifft einen Computer-Tomographen mit einer ein fächerförmiges Strahlenbündel aussendenden Strahlquelle, die um eine Achse rotierbar ist, und einem Strahlendetektor, der von einem diese Achse umgebenden Ring aus einer Reihe von Detektorelementen gebildet ist, sowie mit einem Rechner zur Bildung der Schwächungswerte vorbestimmter Bildpunkte der untersuchten Schicht aus den Ausgangssignalen der Detektorelemente.

Bei einem Computer-Tomographen dieser Art mit feststehenden Strahlendetektor treten (siehe z.B. EP-A-0 133 488) zwei Detektorprobleme auf:

1. Die Anzahl der Detektorelemente ist aus Aufwandsgründen so gering, daß die Bildrekonstruktion nicht während der Gewinnung der Meßdaten erfolgen kann. Die Meßdaten sind für jedes Detektorelement während des Umlaufs der Strahlenquelle zu sammeln und zwischenzuspeichern, so daß eine Real-Time-Meßdatenverarbeitung nicht möglich ist (kein Sofort-Bild).

2. Selbst bei Erhöhung des Hardware-Aufwands durch Verwendung sehr vieler schmaler Detektorelemente würde ein bekannter Computer-Tomograph der genannten Art bei der natürlichen Rekonstruktion mit dem Fokus als Projektionszentrum das Abtast-Theorem verletzen, da das Detektorelement grundsätzlich schmaler als der Detektormittenabstand ist.

Der Erfindung liegt demgemäß die Aufgabe zugrunde, einen Computer-Tomographen der eingangs genannten Art so auszubilden, daß das Abtast-Theorem erfüllt und eine schnelle Bilderzeugung möglich ist.

Diese Aufgabe ist erfindungsgemäß dadurch gelöst, daß der Strahlendetektor während der Rotation der Strahlenquelle um seine Achse drehbar ist. Der Drehwinkel entspricht dabei zweckmäßigerweise $(m : n) \cdot \Delta$, wobei m, n natürliche Zahlen sind, n die Zahl der Umläufe der Strahlenquelle ist und $\Delta$ der Detektormittenabstand ist. Bei der Erfindung erfolgt eine Entkopplung des detektorseitigen Abtastrastermaßes von der physikalischen Detektorbreite, so daß die Erfüllung des Abtast-Theorems ermöglicht ist. Zugleich ermöglicht die Erfindung die Bildrekonstruktion mit dem Fokus als Projektionszentrum, so daß die Real-Time-Meßdatenverarbeitung möglich wird. Somit ist mit der Erfindung auch mit einem Computer-Tomographen der 4. Generation die Erzeugung von Sofort-Bildern möglich.

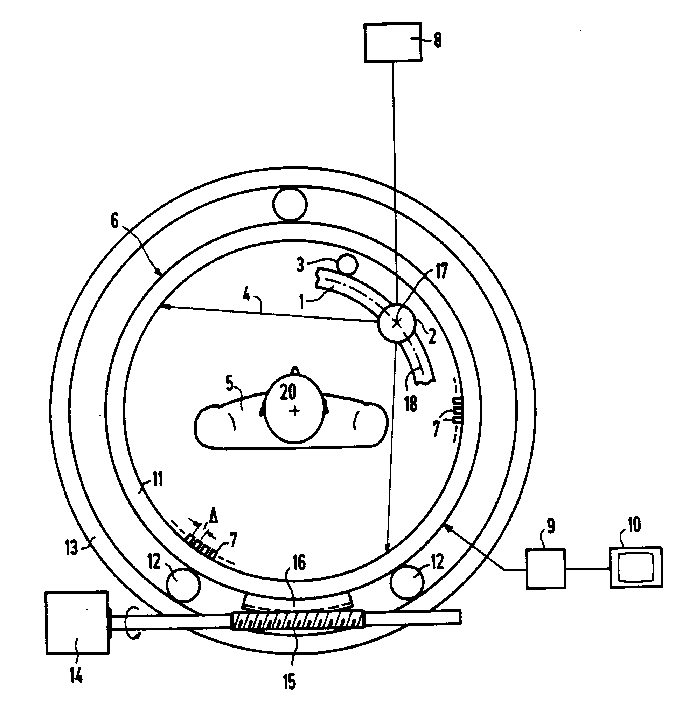

Die Erfindung ist nachfolgend anhand eines in der Zeichnung dargestellten Ausführungsbeispiels näher erläutert.

In der Zeichnung ist ein Computer-Tomograph mit einer auf einem Drehkranz 1 gelagerten Röntgenstrahlenquelle 2 gezeigt, welche mit Hilfe einer Drehvorrichtung 3 um die Achse 20 drehbar ist. Die Röntgenstrahlenquelle 2 sendet ein fächerförmiges Röntgenstrahlenbündel 4 aus, das eine Schicht eines Aufnahmeobjektes 5 aus verschiedenen Richtungen durchsetzt. Die aus dem Aufnahmeobjekt 5 austretende Strahlung wird von einem ringförmigen Strahlendetektor 6 empfangen, der aus einer Reihe von Detektorelementen 7 besteht. Der Detektormittenabstand ist dabei mit $\Delta$ bezeichnet. Die Röntgenstrahlenquelle 2 wird von einem Röntgengenerator 8 gespeist.

Die von den Detektorelementen 7 während des Umlaufs der Röntgenstrahlenquelle 2 gelieferten Daten werden einem Rechner 9 zugeführt, der daraus die Schwächungswerte vorbestimmter Bildpunkte der untersuchten Schicht des Aufnahmeobjektes 5 berechnet und auf einem Sichtgerät 10 bildlich wiedergibt.

Die Detektorelemente 7 sind auf einem Detektorring 11 befestigt, welcher mit Hilfe von Lagern 12 in einem Führungsring 13 um die Achse 20 drehbar gelagert ist. Die Drehung erfolgt durch einen Motor 14 über eine Spindel 15 und einen Zahnkranz 16.

Während der Fokus 17 einmal den ganzen Fokuskreis 18 umläuft, wird der Detektorring 11 um den Bruchteil m/n des Detektormittenabstandes $\Delta$ gedreht. m und n sind dabei natürliche Zahlen, n ist die Anzahl der Umläufe. Wird m zu n teilerfremd gewählt, so wird jede der Detektorelementpositionen im Abstand $\Delta/n$ genau einmal eingenommen, allerdings für m > 1 von verschiedenen Detektorelementen und auch nicht in natürlicher Reihenfolge. Hierzu ein Beispiel:

n = 4 = Zahl der Umläufe des Fokus 17

EP 0 367 856 B1

n = 4 = Zahl der Umläufe des Fokus 17

| k | m 1 | 2 | 3 | |
|---|-----|---|---|---|
| 0 | 0 | 0 | 0 | |
| 1 | 1/4 | 1/2 | 3/4 $\hat{=}$ 3/4 | |
| 2 | 2/4 | 0 | 2/4 $\hat{=}$ 6/4 | |
| 3 | 3/4 | 1/2 | 1/4 $\hat{=}$ 9/4 | |
| 4 | 0 | 0 | 0 $\hat{=}$ 12/4 | |
| 5 | 1/4 | 1/2 | 3/4 $\hat{=}$ 15/4 | |

Tabelle der Detektorelementpositionen

k ist die Nummer des Umlaufs, die Position des Detektorelements ist in Einheiten $\Delta$ angegeben. Für m = 3 erkennt man, daß die Positionen in nicht natürlicher Reihenfolge eingenommen werden und auch von vertauschten Detektorelementen. So wird z.B. die Position 1/4 nach 3 Verschiebungen eingenommen und zwar vom übernächsten Detektorelement.

Von besonderer praktischer Bedeutung ist m = n/2 (n muß hier eine gerade Zahl sein). Das Detektorelement wird bei jedem Umlauf um $\Delta$/2 verschoben. Aber auch m/n = 1/3 bzw. m/n = 2/3 können noch praktisch interessant sein.

Ziel der Erfindung ist die Erfüllung des Abtast-Theorems auf der Detektorseite durch Entkopplung der detektorseitigen Abtastintervallänge $\Delta$ von der physikalischen Detektorelementbreite.

Die Bewegung des Fokus 17 muß synchron mit der Drehung der Spindel 15 erfolgen, um die Bedingung Verschiebung = (m/n)·$\Delta$ pro Fokusumlauf einzuhalten. Der Führungsring 13 kann auch durch eine Lagerung des Ringes 11 in drei Punkten ersetzt werden. Der Fokuskreis 18 darf auch außerhalb des Ringes 11 liegen, wenn dieser z.B. taumelnd gelagert ist.

Es ist auch möglich, die Röntgenstrahlenquelle ohne mechanische Bewegung mit Hilfe von elektrischen oder magnetischen Ablenkvorrichtungen über einen stationären Anodenbogen zu führen.

**Patentansprüche**

1. Computer-Tomograph mit einer ein fächerförmiges Strahlenbündel (4) aussendenden Strahlenquelle (2), die um eine Achse (20) rotierbar ist, und einem Strahlendetektor (6), der von einem diese Achse umgebenden Ring (11) mit einer Reihe von Detektorelementen (7) gebildet ist, sowie mit einem Rechner (9) zur Bildung von Schwächungswerten vorbestimmter Bildpunkte einer untersuchten Schicht aus Ausgangssignalen der Detektorelemente (7), **dadurch gekennzeichnet,** daß während der Rotation der Strahlenquelle (2) der Strahlendetektor (6) um einen Drehwinkel um seine Achse (20) drehbar ist.

2. Computer-Tomograph nach Anspruch 1, **dadurch gekennzeichnet,** daß der Drehwinkel $\frac{m}{n}$ ·$\Delta$ entspricht, wobei m, n natürliche Zahlen sind, n die Zahl der Umläufe der Strahlenquelle (2) ist und $\Delta$ der Detektormittenabstand ist.

3. Computer-Tomograph nach Anspruch 2, **dadurch gekennzeichnet,** daß m = $\frac{n}{2}$ und n eine gerade

3

Zahl ist.

4. Computer-Tomograph nach Anspruch 2, **dadurch gekennzeichnet,** daß $\frac{m}{n} = \frac{1}{3}$ ist.

5. Computer-Tomograph nach Anspruch 2, **dadurch gekennzeichnet,** daß $\frac{m}{n} = \frac{2}{3}$ ist.

## Claims

1. A computer tomograph with an x-ray source (2) emitting a fan-shaped beam of rays (4), which is able to be rotated about an axis (20), and with a ray detector (6), which is formed by a ring (11) surrounding this axis with a series of detector elements (7), as well as with a computer (9) for forming attenuation values of predetermined pixels of an examined layer from the output signals of the detector elements (7), characterised in that during the rotation of the x-ray source (2) the ray detector (6) is able to be rotated about its axis by an angle of rotation.

2. A computer tomograph according to claim 1, characterised in that the rotary angle corresponds to $\frac{m}{n} \cdot \Delta$, wherein m, n are natural numbers, n is the number of revolutions of the x-ray source (2) and $\Delta$ the detector centre-to-centre spacing.

3. A computer tomograph according to claim 2, characterised in that m $= \frac{n}{2}$ and n is an even number.

4. A computer tomograph according to claim 2, characterised in that $\frac{m}{n} = \frac{1}{3}$ .

5. A computer tomograph according to claim 2, characterised in that $\frac{m}{n} = \frac{2}{3}$ .

## Revendications

1. Tomodensitomètre assisté par ordinateur, comportant une source de rayonnement (2), qui émet un faisceau de rayonnement (4) en éventail et qui peut tourner autour d'un axe (20), et par un détecteur de rayonnement (6), qui est formé par un anneau (11) entourant cet axe et qui comporte une série d'éléments de détection (7), ainsi qu'un ordinateur (9) servant à former l'image de valeurs d'affaiblissement de points images prédéterminés d'une couche examinée, à partir des signaux de sortie des éléments de détection (7), caractérisé par le fait que pendant la rotation de la source de rayonnement (2), le détecteur de rayonnement (6) peut tourner sur un angle de rotation autour de son axe (20).

2. Tomodensitomètre assisté par ordinateur, selon la revendication 1, caractérisé par le fait que l'angle de rotation correspond à
$\frac{m}{n} \cdot \Delta$, m, n étant des nombres entiers naturels, n représentant le
nombre des rotations de la source de rayonnement (2) et $\Delta$ la distance entre les centres des détecteurs.

3. Tomodensitomètre assisté par ordinateur, suivant la revendication 2,
caractérisé par le fait que l'on a m $= \frac{n}{2}$ et que n est un nombre entier.

4. Tomodensitomètre assisté par ordinateur, suivant la revendication 2,
caractérisé par le fait que l'on a $\frac{m}{n} = \frac{1}{3}$ .

5. Tomodensitomètre assisté par ordinateur, suivant la revendication 2,
caractérisé par le fait que l'on a $\frac{m}{n} = \frac{2}{3}$ .